Europäisches Patentamt

European Patent Office

Office européen des brevets

(19)

(11) Publication number: **0 025 467**
**B1**

# (12) EUROPEAN PATENT SPECIFICATION

(45) Date of publication of patent specification: **11.05.83**

(51) Int. Cl.³: **C 12 Q 1/04, C 12 Q 1/12**

(21) Application number: **79400638.7**

(22) Date of filing: **12.09.79**

(54) Chromogenic chemical substrates for identification of microbial colonies.

(43) Date of publication of application:
**25.03.81 Bulletin 81/12**

(45) Publication of the grant of the patent:
**11.05.83 Bulletin 83/19**

(84) Designated Contracting States:
**CH DE FR GB NL SE**

(56) References cited:
CHEMICAL ABSTRACTS, vol. 85, no. 11,
September 13 1976, page 233, abstract
74277x. Columbus, Ohio, USA
CHEMICAL ABSTRACTS, vol. 78, no. 25, June
25, 1973, page 172 abstract 156487k.
Columbus, Ohio, USA Novel G. et al. "Mutants
of Escherichia coli K12 unable to grow on
methyl-beta-D-glucuronide. Map Location of uid
A locus of the structural gene of beta-D-
glucuronidase"
CHEMICAL ABSTRACTS, vol. 86, no. 25, June
20, 1977, page 268, abstract 185673p.
Columbus, Ohio, USA MANDRAND-
BERTHELOT, M. A. "Gratuitous induction of
Escharichia Coli K12 beta-glucuronidase and its
double mechanism of repression"

(73) Proprietor: **Rambach, Alain**
**75, rue Madame**
**F-75006 Paris (FR)**

(73) Proprietor: **Société TECHNOGRAM**
**19, rue Théodore Deck**
**F-75015 Paris (FR)**

(72) Inventor: **Rambach, Alain**
**75, rue Madame**
**F-75006 Paris (FR)**

(74) Representative: **Martinet, René et al,**
**Cabinet Martinet 62, rue des Mathurins**
**F-75008 Paris (FR)**

(56) References cited:
CHEMICAL ABSTRACTS, vol. 42, no. 21,
November 10, 1948, abstract 8913c Columbus,
Ohio, USA FRIEDENWALT et al. "The
histochemical localization of glucuronidase"·
CHEMICAL ABSTRACTS, vol. 81, no. 21,
November 25, 1974, page 454, abstract
136459m. Columbus, Ohio, USA
Biochimie, 1977, 59, 163—170

Courier Press, Leamington Spa, England.

Chromogenic chemical substrates for identification of microbial colonies

Field of the invention

The present invention concerns chromogenic chemical substrates permitting recognition of *Escherichia coli* colonies by incorporation of the chemical substrates into the growth medium. The identification is obtained from the specific coloration of the colonies.

*Escherichia coli* is with few exceptions found in the fecal wastes of all warm-blooded animals. This characteristic, along with its simple nutritional requirements, makes this organism a well-chosen candidate as an indicator of fecal contamination. A simple method and means for chromogenically detecting *Escherichia coli* colonies is therefore of practical importance.

Description of the prior art

Chromogenic substrates are already used to identify enzymes and examples of such known substrates will be given in the following. However, these substrates are inadapted to certain microbial identifications on colonies.

In certain cases, the coloration specific for the particular enzyme does not appear spontaneously after the enzymatic reaction on the substrate, since the degradation product is colorless and it is necessary to further add a developer in oder to cause the color to appear. As a result, not only another dispensation of liquid (the developer) has to be made, but it is impossible to continually observe the reaction to be studied.

Other substrates proposed in the prior art liberate colored products volatile or diffusible and this limits the time during which the solution or the biological suspension can be left in the presence of the substrate before important problems of diffusion or evaporation are encountered.

Finally, other substrates previously proposed become yellow after the enzymatic reaction, which is a color difficult to detect and to visualize, as many biological media already have this color per se.

Jonas S. Friedenwald and Bernard Becker "The Histochemical Localization of Glucuronidase", J. Cell. and Comp. Physiol., 31, 303—310, 1948, have developed a sensitive photocolorimetric method for the quantitative assay of glucuronidase activity. The tissue to be tested is incubated with 8-hydroxyquinoline glucuronide in the presence of ferric ions in a solution saturated with ferric hydroxyquinoline. The enzymatically liberated 8-hydroxyquinoline is precipitated as ferric hydroxyquinoline at the presumption site of the glucuronidase activity. This black precipitate is then converted into Prussian Blue.

Further it is known *Escherichia coli* has the capability of synthetizing the glucuronidase enzymatic activity. One could thus expect that, if 8-hydroxyquinoline-glucuronide is incorporated in a microbiological medium in the presence of ferric ions in which *Escherichia coli* is caused to grow, the corresponding colonies would be detected in the form of black blobs.

Unfortunately, these black blobs are neither formed nor observed by the bacteriologist when he treats *Escherichia coli* in the presence of 8-hydroxyquinoline glucuronide reagent and ferric ions.

Summary of the invention

I have found that, by supplementing the 8-hydroxyquinoline glucuronide reagent with an activator in the presence of ferric ions, the *Escherichia coli* colonies give rise to black blobs.

This activator is X-glucuronide where X is a radical selected from methyl, benzyl, phenyl, the halogeno and nitro substitutes of these radicals.

Preferably X is halogenomethyl, nitromethyl, halogenobenzyl, nitrobenzyl, halogenophenyl or nitrophenyl.

The colored product of degradation is insoluble. This is most useful when, for example, several microorganisms develop on the same solid medium and give rise to colonies well separated from each other, because the colored product does not diffuse from one colony to the other. It is thus possible to see directly by the color of the colony whether the corresponding microorganism is *Escherichia coli* or not.

Another advantage of the chromogenic substrates according to the invention is that they can withstand 120°C so that the microbiological medium which contains them can be sterilized by simple autoclaving without disparition of the chromogenicity.

Brief description of the drawing

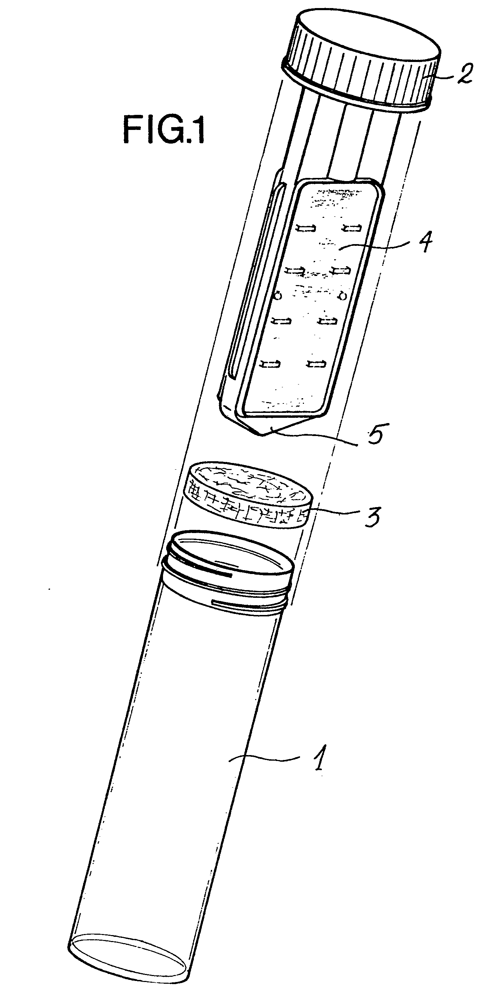

Fig. 1 is a perspective view of a commercially available kit for detecting bacterial contamination by *Escherichia coli*.

Description of the preferred embodiments

A test kit commonly used for detecting microbiological contamination in liquids and which can be used for putting the invention into practice is shown in Fig. 1. It consists of a sterile container 1 with a pad 5 connected to the cap 2 of the container. The pad 5 has a cavity filled with a nutrient 4. Absorbent cotton 3 is located at the bottom of the container for absorbing excess liquid. The pad is immersed in the test liquid and returned to its container to be incubated for the appropriate time and temperature. The nutrient medium 4 has incorporated thereto the reagent and activator previously defined.

First example

The reagent is 8-hydroxyquinoline-$\beta$ -D glucuronide in the presence of ferric salts and the activator is phenyl-$\beta$-D glucuronide

The degradation product is ferric hydroxyquinoline which is black and insoluble.

Second example
The reagent is as in the first example, except the activator is bromophenyl-β-D glucuronide

Third example
The microbiological control of liquids is often performed with the aid of a sampler containing a solid nutrient medium on which the microorganisms present in the liquid can grow and form colonies visible to the naked eye. It is important to know whether fecal bacteria are present. The former procedure is to detect whether bacteria, capable of fermenting lactose and growing at exactly 44.5°C, are present.

Instead of this procedure which requires a very precise temperature of growth, thus a delicate instrument, one can use according to the invention a much simpler procedure. It consists in taking as reagent a solution of 8-hydroxyquinoline-β-D glucuronide at 1/1000 containing ammoniacal ferric citrate and as activator nitrophenyl-β-glucuronide at a concentration of 500 μg. per ml.

Since precise temperature, 44.5°C, is no longer necessary in this new procedure, the user of a pool can simply dip a sampler containing a medium ready to use into the pool water and let the sampler incubate either in the sun or in any lukewarm place to see whether black colonies appear, thus indicating the presence of fecal bacteria. In a more general fashion this approach permits systematic control of the presence or absence of bacteriological contamination of water, and environment, without requiring costly instruments sometimes difficult to transport to the sites.

Claims

1. Chromogenic substrate member for identifying Escherichia coli bacteria comprising:
a growing medium for said bacteria;
container means for said growing medium and a liquid sample to be tested; said container means also containing:
a reagent comprising 8-hydroxyquinoline-β-D glucuronide and ferric ions, and it is characterized in that it further comprises:
a chromogenic activator compound consisting in X-glucuronide where X is selected from methyl, benzyl, or phenyl, the halogeno substitutes and nitro substitutes thereof whereby Escherichia coli bacteria form dark pigmented blobs on said medium.
2. Chromogenic substrate member according to claim 1, characterized in that the activator compound is: methyl-glucuronide
3. Chromogenic substrate member according to claim 1, characterized in that the activator compound is: a halogeno-methyl-glucuronide
4. Chromogenic substrate member according to claim 1 characterized in that the activator compound is: a nitro-methyl-glucuronide
5. Chromogenic substrate member according to claim 1 characterized in that the activator compound is: benzyl-glucuronide
6. Chromogenic substrate member according to claim 1 characterized in that the activator compound is: a halogeno-benzyl-glucuronide
7. Chromogenic substrate member according to claim 1 characterized in that the activator compound is: a nitro-benzyl-glucuronide
8. Chromogenic substrate member according to claim 1 characterized in that the activator compound is: phenyl-glucuronide
9. Chromogenic substrate member according to claim 1 characterized in that the activator compound is: a halogeno-phenyl-glucuronide
10. Chromogenic substrate member according to claim 1 characterized in that the activator compound is: a nitro-phenyl-glucuronide

Revendications

1. Dispositif formant substrat chromogénique pour l'identification des bactéries Escherichia coli comprenant:
un milieu de croissance pour lesdites bactéries;
des moyens destinés à contenir ledit milieu de croissance et un liquide échantillon à tester, lesdits moyens contenant également:
un réactif comprenant de la 8-hydroxy-quinoléine-β-D glucuronide et des ions ferriques, et il est caractérisé en ce qu'il comprend en outre
un composé d'activation chromogénique consistant en X-glucuronide où X est choisi parmi les radicaux méthyle, benzyle ou phényle, leurs substitués halogénés ou leurs substitués nitrés.
d'où il résulte que les bactéries Escherichia coli forment dans ledit milieu des taches pigmentées noires.
2. Dispositif formant substrat chromogénique conforme à la revendication 1, caractérisé en ce que le composé activateur est le: méthyl-glucuronide
3. Dispositif formant substrat chromogénique conforme à la revendication 1, caractérisé en ce que le composé activateur est un: halogéno-méthyl-glucuronide
4. Dispositif formant substrat chromogénique conforme à la revendication 1, caractérisé en ce que le composé activateur est un: nitro-méthyl-glucuronide
5. Dispositif formant substrat chromogénique conforme à la revendication 1, caractérisé en ce que le composé activateur est le: benzyl-glucuronide
6. Dispositif formant substrat chromogénique conforme à la revendication 1, caractérisé en ce que le composé activateur est un: halogéno-benzyl-glucuronide
7. Dispositif formant substrat chromogénique conforme à la revendication 1, caractérisé en ce que le composé activateur est un: nitro-benzyl-glucuronide

8. Dispositif formant substrat chromogénique conforme à la revendication 1, caractérisé en ce que le composé activateur est le: phényl-glucuronide

9. Dispositif formant substrat chromogénique conforme à la revendication 1, caractérisé en ce que le composé activateur est un: halogéno-phényl-glucuronide

10. Dispositif formant substrat chromogénique conforme à la revendication 1, caractérisé en ce que le composé activateur est un: nitro-phényl-glucuronide

**Patentansprüche**

1. Chemisches chromogenes Substrat zur Identifizierung von Escherichia Colibakterien, mit einem Nährboden für diese Bakterien und einen Behälter für diesen Nährboden und eine zu prüfende Flüssigkeitsprobe, wobei der Behälter außerdem eine Reagenz mit 8-Hydroxyquinoline-$\beta$-D-Glucuronid und Eisenionen enthält, dadurch gekennzeichnet, daß es außerdem einen chromogenen Aktivator aus X-Glucuronid, mit X ausgewählt aus Methyl, Benzyl oder Phenyl, den Halogeno-Substituenten und Nitro-Substituenten dazu, aufweist, wodurch Eschericha Colibakterien dunkelpigmentierte Klumpen auf diesem Medium bilden.

2. Chemisches chromogenes Substrat nach Anspruch 1, dadurch gekennzeichnet, daß der Aktivator aus Methyl-Glucuronid besteht.

3. Chemisches chromogenes Substrat nach Anspruch 1, dadurch gekennzeichnet, daß der Aktivator ein Halogeno-Methyl-Glucuronid ist.

4. Chemisches chromogenes Substrat nach Anspruch 1, dadurch gekennzeichnet, daß der Aktivator ein Nitro-Methyl-Glucuronid ist.

5. Chemisches chromogenes Substrat nach Anspruch 1, dadurch gekennzeichnet, daß der Aktivator ein Benzyl-Glucuronid ist.

6. Chemisches chromogenes Substrat nach Anspruch 1, dadurch gekennzeichnet, daß der Aktivator ein Halogeno-Benzyl-Glucuronid ist.

7. Chemisches chromogenes Substrat nach Anspruch 1, dadurch gekennzeichnet, daß der Aktivator ein Nitro-Benzyl-Glucuronid ist.

8. Chemisches chromogenes Substrat nach Anspruch 1, dadurch gekennzeichnet, daß der Aktivator ein Phenyl-Glucuronid ist.

9. Chemisches chromogenes Substrat nach Anspruch 1, dadurch gekennzeichnet, daß der Aktivator ein Halogeno-Phenyl-Glucuronid ist.

10. Chemisches chromogenes Substrat nach Anspruch 1, dadurch gekennzeichnet, daß der Aktivator ein Nitro-Phenyl-Glucuronid ist.

# FIG.1